# EUROPÄISCHE PATENTSCHRIFT

(11) **EP 1 440 118 B1**
(45) Veröffentlichungstag und Bekanntmachung des Hinweises auf die Patenterteilung: **01.06.2005**
(21) Anmeldenummer: 02785189.8
(22) Anmeldetag: 10.10.2002
(51) Int. Cl.: C08L 23/10, C08L 53/02, C07D 401/14, C08L 51/00, C09K 15/30, C08K 5/3492, C08L 53/00, C08K 5/00, C08G 73/02, C08L 55/02, C08K 5/098, C08G 73/06, C08K 5/20, C08L 23/16, C08L 23/02, C08L 67/02, C08J 3/00, C08F 10/06, C08L 91/08, C08L 21/00, C08L 23/22

(54) **MISCHUNGEN AUS WACHSEN UND POLYMERADDITIVEN**
BLENDS OF WAXES AND POLYMER ADDITIVES
MELANGES DE CIRES ET D'ADDITIFS POLYMERES

(30) Priorität: 20.10.2001 DE 10152228
(43) Veröffentlichungstag der Anmeldung: 28.07.2004
(73) Patentinhaber: Clariant GmbH, 65929 Frankfurt am Main (DE)
(72) Erfinder: RICHTER, Eric, 86672 Thieraupten (DE); LECHNER, Christian, 86857 Hurlach (DE); BOTT, Rainer, 25337 Seeth-Ekholt (DE)
(74) Vertreter: Paczkowski, Marcus, Dr.
(86) Internationale Anmeldenummer: PCT/EP2002/011334
(87) Internationale Veröffentlichungsnummer: WO 2003/035745

(56) Entgegenhaltungen:
- WO-A-00/32695
- WO-A-02/46300
- WO-A-98/54175
- WO-A-98/54176
- WO-A-98/54177
- DE-A- 19 859 096
- US-A- 3 705 123
- US-A- 4 914 145

## Beschreibung

Die Erfindung betrifft Mischungen aus Wachsen und Polymeradditiven und ihre Verwendung.

Die Verarbeitung von Kunststoffen erfolgt bis auf wenige Ausnahmen in der Schmelze. Damit verbunden sind Struktur- und Zustandsänderungen, die kaum ein Kunststoff übersteht, ohne sich in seiner chemischen Struktur zu verändern. Vernetzungen, Oxidation, Molekulargewichtsänderungen und damit auch Änderungen der physikalischen und technischen Eigenschaften können die Folge sein. Um die Belastung der Polymere während der Verarbeitung zu reduzieren, setzt man je nach Kunststoff unterschiedliche Additive ein.

Aus diesem Grund werden einigen Kunststoffen Stabilisatoren zugesetzt, die die Veränderungsprozesse wie Vemetzungs- oder Abbaureaktionen unterbinden oder zumindest bremsen. Weiterhin werden den meisten Kunststoffen in nicht unerheblichen Maße Gleitmittel, insbesondere Montanwachse, beigemengt, welche die Aufgabe haben, das Fließverhalten der Schmelze zu verbessern. Dies wird erreicht, in dem die Gleitmittel die innere und äußere Reibung reduzieren und somit die mechanochemische Schädigung des Materials herabsetzen.

Heute werden in der Regel eine Vielzahl unterschiedlicher Additive gleichzeitig eingesetzt, die jedes für sich eine Aufgabe übernimmt. So werden Antioxidantien und Stabilisatoren eingesetzt, damit der Kunststoff ohne chemische Schädigung die Verarbeitung übersteht und anschließend lange Zeit gegen äußere Einflüsse wie Hitze, UV-Licht, Witterung und Sauerstoff (Luft) stabil ist.

Gleitmittel sind dafür verantwortlich, dass ein Kunststoff in der Verarbeitungsmaschine gezielt und ausreichend aufschließt und als homogene Schmelze vorliegt, sie verhindern ein zu starkes Kleben der Kunststoffschmelze an heißen Maschinenteilen, verbessern die Fließeigenschaften der Kunststoffschmelze und wirken als Dispergiermittel für Pigmente, Füllstoffe und Verstärkungsstoffe.

Stand der Technik ist der Einsatz von unterschiedlichen Wachsen als Entformungs- und Trennmittel in Kunststoffen, insbesondere in Polyamiden. Aufgrund der hohen Verarbeitungs- und Gebrauchstemperaturen und der hohen Reaktivität der Polyamide ist die Auswahl an möglichen Additiven allerdings stark begrenzt. Zu den am häufigsten eingesetzten Produktgruppen gehören Metallstearate, Amidwachs und Montanwachsester, die je nach Anforderungsprofil als unterschiedliche Produkte eingesetzt werden. Alle diese drei Produktgruppen wirken als Trenn- und Entformungsmittel.

Die Metallstearate verbessern gleichzeitig die Fließfähigkeit der Schmelze. Diese wird jedoch, vor allem durch das hygroskopische Verhalten von Polyamid verursacht, durch einen nicht unerheblichen Molekulargewichtsabbau des Polymeren hervorgerufen. Hingegen verbessern die Montanwachsester und deren Salze die Fließfähigkeit von Kunststoffen Polyamid allein durch innere Gleitwirkung, ohne das Molekulargewicht des Polymeren zu reduzieren.

Durch die hohen Verarbeitungstemperaturen sowie die chemische Aggressivität der Schmelze gegenüber Farbmitteln stellt die Einfärbung von speziell Polyamid besonders hohe Anforderungen.

Die Auswahl in Frage kommender Farbmittel ist ebenso eingeschränkt wie die geeigneter Dispergiermittel. Auch thermostabile Pigmente gegriffen werden durch die zusätzliche hohe Scherenergie bei der Dispergierung häufig nachhaltig geschädigt. Belastbare Pigmente sind häufig wirtschaftlich nicht in größeren Mengen erhältlich, so dass versucht wird, entsprechend die Dispergierhilfsmittel zu optimieren.

Hierbei haben sich seit Jahrzehnten Montanwachse bewährt. Sie bieten durch ihre Gleitwirkung die Möglichkeit empfindliche Pigmente (allein oder in Kombination mit anderen organischen oder anorganischen Pigmenten, Additiven, Füllstoffen) schonend einzuarbeiten, sind andererseits aber auch hinreichend thermisch belastbar, gut mit der Polyamidschmelze verträglich und auch bei der weiteren Verarbeitung unproblematisch. Gleiches gilt für Modifikationen, die auf Montanwachsbasis hergestellt und zielgerichtet optimiert wurden.

Neben der ausschließlichen Verwendung von Montanwachsen als Dispergierhilfsmittel für Pigmente können ebenso unterschiedliche Amidwachse eingesetzt werden. Teilweise lassen sich auch Kombinationen aus beiden finden, um z.B. die dispergierfördemde Wirkung des Montanwachses zu nutzen, die Rezepturkosten aber durch ein billigeres Produkt (Amidwachs) zu reduzieren.

Insbesondere wenn ein Polyamid-Farbkonzentrat zur Herstellung faserverstärkter Teile verwendet werden soll, bekommt die Stabilität des Konzentrates sowie natürlich seiner Einzelkomponenten besondere Bedeutung: Durch die erhöhte Schererwärmung (zusätzlich zur ohnehin hohen Verarbeitungstemperatur) können Farbmittel in der Endanwendung geschädigt werden, obwohl sie den Compoundier- bzw. Dispergierschritt (noch) unbeschadet überstanden haben. Üblicherweise versucht man durch nachträgliche Prozessstabilisierung das Problem in den Griff zu bekommen. Allerdings wird bei nachträglicher Stabilisierung häufig keine ausreichende Homogenisierung erreicht, besonders dann wenn ein pulverförmiger Stabilisator dem Kunststoffgranulat zugegeben wird.

Während der Verarbeitung von vielen Kunststoffen, insbesondere den Polyamiden, herrschen sehr hohe Temperaturen, die das Polymere schädigen. Weiterhin hat die lange Einwirkung von Luft, Licht und Wärme einen negativen Einfluss auf die Eigenschaften des Kunststoffs. Um Schädigung während der Verarbeitung und während der Nutzungsdauer zu unterdrücken werden viele Kunststoffe stabilisiert. Für die unterschiedlichen Anwendungen haben sich insbesondere Kupfersalze, aromatische Amine und sterisch gehinderte Phenole in unterschiedlichen Kombinationen bewährt. Bekannt ist ebenfalls N,N'-bis(2,2,6,6-tetramethyl-4-piperidyl)-1,3-benzoldicarboxamid als multifunktionales Additiv (Verfahrens- und Hitzestabilisator) für Polyamide.

So wird die Verwendung von verschiedenen Wachsen etwa in der WO 00/32695 beschrieben. Hierbei handelt es sich insgesamt um eine Polymer-Zusammensetzung, die
(1) ein Polymer
(2) einen Stabilisator
(3)
   (a) natürliche Wachse
   (b) Polyolefinwachse oder Paraffinwachse
   (c) Metalloxide oder ähnliche Verbindungen
umfasst.

Hiermit wird die Aufgabe, geeignete Verschlüsse für Getränkeflaschen zur Verfügung zu stellen, dadurch gelöst, dass diese Verschlüsse aus einem Polymer hergestellt werden, welches u.a. Wachse in äußerst geringen Mengen enthält.

Die vorgenannten Additive können in den verschiedensten Verfahrensschritten in den Kunststoff eingebracht werden. So ist es bei Polyamiden möglich, bereits am Ende der Polykondensation oder in einem folgenden Compoundierprozess die Wachse in die Polyamidschmelze einzumischen. Weiterhin gibt es Verarbeitungsprozesse bei denen die Wachse erst später zugefügt werden. Dies wird insbesondere beim Einsatz von wachshaltigen Pigment- oder Additivmasterbatches praktiziert. Außerdem besteht die Möglichkeit, insbesondere pulverförmige Wachse auf das durch den Trockenprozess eventuell warme Polyamidgranulat aufzutrommeln.

Nach dem bisher bekannten Stand der Technik sind die Wirkungen der verschiedenen aufgeführten (Hilfs-)Mittel in bezug auf eine Reihe von Eigenschaften, wie beispielsweise innere, äußere Gleitwirkung sowie Dispergierung von Pigmenten und Füllstoffen und Glanz des Kunststoffes, noch nicht ausreichend.

Es war daher Aufgabe der vorliegenden Erfindung, solche (Hilfs-)Mittel zur Verfügung zu stellen, die eine insgesamt verbesserte Wirkung auf den Kunststoff, insbesondere bei Polyamiden, ausüben.

Gelöst wird diese Aufgabe durch eine Mischung aus einem Wachs (Komponente A) und einem Polymeradditiv (Komponente B), dadurch gekennzeichnet, dass es sich bei der Komponente A um Umsetzungsprodukte von Montanwachssäuren mit Ethylenglykol und/oder um Umsetzungsprodukte von Montanwachssäuren mit einem Calciumsalz und es sich bei der Komponente B um N,N'-bis-pyperidyl-1,3-benzoldicarboxamid oder um N,N'-bis(2,2,6,6-tetramethyl-4-piperidyl)-1,3-benzoldicarboxyamid handelt und wobei die Mischung 10 bis 90 Gew.-% an Komponente A und 90 bis 10 Gew.-% an Komponente B enthält.

Überraschenderweise wurde gefunden, dass eine Kombination aus Montanwachsen und bestimmter Polymeradditiven durch synergistische Effekte insbesondere in Polyamiden die besten Ergebnisse in Bezug auf innere, äußere Gleitwirkung sowie Dispergierung von Pigmenten und Füllstoffen und Glanz, verglichen zur Verwendung der Einzelsubstanzen sowie einer Reihe weiterer Wachse liefert.

Bevorzugt handelt es sich bei den Umsetzungsprodukten um eine Mischung aus Ethylenglykol-Mono-Montanwachssäureester, Ethylenglykol-dimontanwachssäureester, Montanwachssäuren und Ethylenglykol.

Besonders bevorzugt es sich bei den Umsetzungsprodukten um eine Mischung aus 1,3-Budandiol-Mono-Montanwachssäureester, 1,3-Budandiol-Di-Montanwachssäureester, Montanwachssäuren, 1,3-Butandiol, Calciummontanat, und dem Calciumsalz.

Bevorzugt enthält die Mischung 30 - 70 Gew.-% an Komponente A und 70 bis 90 Gew.-% an Komponente B.

Besonders bevorzugt enthält die Mischung 45 bis 55 Gew.-% an Komponente A und 55 bis 45 Gew.-% an Komponente B.

Bevorzugt liegen die Komponenten A und B als Granulat, Schuppen, Feinkorn, Pulver und/oder Micronisat vor.

Bevorzugt liegen die Komponenten A und B als Granulat, Schuppen, Feinkorn, Pulver und/oder Micronisat vor.

Bevorzugt liegen die Komponenten A und B als physikalische Mischung der Feststoffe, als Schmelzmischung, als Kompaktat oder in Form eines Masterbatches vor.

Die Erfindung betrifft auch die Verwendung der erfindungsgemäßen Mischung als Gleit-, Trenn- und/oder Dispergiermittel.

Bevorzugt wird die Mischung in einer Formmasse eines Polykondensats verwendet.

Bevorzugt handelt es sich bei dem Polykondensat um Polyamid.

Bevorzugt handelt es sich bei den Polyamiden um solche vom Aminosäure-Typ und/oder vom Diamin-Dicarbonsäure-Typ.

Bevorzugt handelt es sich bei den Polyamiden um Polyamid 6 und/oder Polyamid 66.

Bevorzugt sind die Polyamide unverändert, gefärbt, gefüllt, ungefüllt, verstärkt, unverstärkt oder auch anders modifiziert.

Bevorzugt werden die Komponenten A und B an den gleichen oder unterschiedlichen Prozessschritten bei der Herstellung/Verarbeitung von Polyamiden eingebracht.

Bevorzugt erfolgt die Einarbeitung der Komponente A und B in der Polykondensation, in der Compoundierung oder direkt bei der formgebenden Verarbeitung.

Bevorzugt beträgt die Gesamtmenge der Komponenten A und B 0,01 bis 10,00 Gew.-% im Polykondensat.

Besonders bevorzugt beträgt die Gesamtmenge der Komponenten A und B 0,1 bis 2,00 Gew.-% im Polykondensat.
Geeignete Gleitmittel stammen aus der Gruppe der Montanwachse. Die Montanwachse gehören chemisch zu den Säure- und Esterwachsen. Sie lassen sich ganz allgemein als eine Mischung einer langkettigen linearen gesättigten Carbonsäure, mit unterschiedlichen Estern dieser Carbonsäure mit verschiedenen Alkoholen und den Salzen der Carbonsäuren beschreiben. In aller Regel enthalten diese Produkte weiterhin sogenanntes natives Montanwachs, was den Ester eine langkettigen Carbonsäure mit einem langkettigen monofunktionellen Alkohol darstellt. Die in Montanwachsen enthaltenen Carbonsäuren haben eine Kettenlänge von 24 bis 36 Kohlenstoffatomen.

Im Bereich der Kunststoff-Industrie spielen verschiedene Produkte eine kommerzielle Rolle. So werden vor allem die Ester der Montanwachssäure mit Glykol, Glycerin, Butandiol (1,3- und 1,4-), Trimethylolpropan, Pentaerythrit und Dipentaerythrit eingesetzt. Weiterhin finden deren teilverseiften Produkte Verwendung. All diese Produkte zeichnen sich dadurch aus, dass ihre Verseifungszahl, ihre Säurezahl und gegebenenfalls ihr Metallanteil in weiten Bereichen variiert werden kann und somit die Produkte an die Anwendung angepasst werden können.

Bei den geeigneten Montanwachsen handelt es sich um das Umsetzungsprodukt von Montanwachssäure bzw. einer Mischung von nativen Montanwachsestern und Montanwachssäuren mit Ethylenglycol zu einer Mischung des Mono- und Diesters und der Edukte (z.B. ®Licowax E, Clariant GmbH). Besonders geeignet ist auch ist das Calciumsalz der Montanwachssäuren. Dieses Produkt ist ein Umsetzungsprodukt der Montanwachssäure bzw. einer Mischung von nativen Montanwachsestem und Montanwachssäuren mit einem Calciumsalz zu einer Mischung aus Calciummontanat und den Edukten (z.B. ® Licomont CaV 102, Clariant GmbH).

Bei den geeigneten Polymeradditiven handelt es sich um Verbindungen des Typs der aromatischen Di- oder Tri-carboxylester bzw. -amide. Insbesondere hat sich herausgestellt, dass substituierte Substanzen des Typs N,N'-bis pyperidyl-1,3-benzodicarboxamid geeignet sind, beispielsweise das N,N'-bis(2,2,6,6-tetramethyl-4-piperidyl)-1,3-benzodicarboxamid (Nylostab® S-EED, Clariant GmbH).

So ist die innere sowie äußere Gleitwirkung der erfindungsgemäßen Mischung aus einem Wachs (Komponente A) und einem Polymeradditiv (Komponente B) im Vergleich zur Verwendung herkömmlicher Wachse bei gleicher Gesamtkonzentration deutlich höher. Durch den kombinierten Einsatz der beiden Komponenten, d.h. der Mischung erhöht sich Fließfähigkeit des Compounds deutlich im Vergleich zu den Einzelsubstanzen. Gleichzeitig zeichnet sich diese Kombination durch eine sehr starke Trennwirkung aus. Die Klebeneigung der Polyamid-Formmasse an heißen Maschinenteile wird deutlich reduziert.

Weiterhin werden die optischen Eigenschaften eine Polyamid-Formteils (Oberflächenglanz) durch den Einsatz einer erfindungsgemäßen Mischung aus einem Wachs (Komponente A) und einem Polymeradditiv (Komponente B) deutlich verbessert.

In Pigmentmasterbatchen mit Polyamid als Trägermaterial führt der Einsatz der erfindungsgemäßen Mischung aus einem Wachs (Komponente A) und einem Polymeradditiv (Komponente B) zur Verbesserung der Eigenschaften, insbesondere einer feineren und gleichmäßigeren Verteilung von Pigmenten und Füllstoffen. Diese verbesserte Dispergierwirkung wird durch deutlich niedrigere Filterdruckwert sowie durch eine erhöhte Farbstärke messtechnisch erfasst.

Dieser beobachtete Synergismus bei der erfindungsgemäßen Mischung aus einem Wachs (Komponente A) und einem Polymeradditiv (Komponente B) ist überraschend, da aufgrund der chemischen Strukturen und der bekannten Wirkmechanismen keinerlei Wechselwirkungen zwischen diesen Wachsen und Polymeradditiven zu erwarten sind.

Die Einarbeitung der pulverförmigen bzw. feinkomartigen Substanzen erfolgt durch Einmischen bzw. Aufziehen der Substanzen auf das kalte oder warme Trägerpolymer und anschließende Verarbeitung durch einen formgebenden Verarbeitungsschritt (z.B. Spritzguss, Flachfolienhersteflung, Kalandrieren, etc.) bzw. durch vorheriges Eincompoundieren mittels eines Extruders, wobei beide Komponenten mittels Seitendosierung dosiert oder zuvor in das Polyamid eingemischt werden können. Hier sind einfache physikalische Mischungen der Feststoffe, Schmelzmischungen oder Kompaktate möglich.

### Trennwirkung (äußere Gleitwirkung)

Gleitmittel werden in der Kunststoffindustrie als äußere Gleitmittel eingesetzt, um ein Anhaften der heißen Kunststoffschmelze an die heißen Metalloberflächen der Verarbeitungsmaschine zu verhindern. Sie bilden einen Gleitfilm zwischen der Metalloberfläche und der Polymerschmelze und fungieren hier als Trennmittel.

Die Trennwirkung (äußere Gleitwirkung) von Gleitmitteln in technischen Kunststoffen wie Polyamid wird üblicherweise durch die Entformungskraftmessung beim Spritzgießen quantifiziert. Dazu wird eine zylindrische Hülse im Spritzgießverfahren hergestellt und die benötigte Maximalkraft zum Entformen der Hülse aus dem Werkzeug als Entformungskraft registriert. Je niedriger die Entformungskraft, desto besser ist die äußere Gleitwirkung des eingesetzten Gleitmittels.

Für die Untersuchungen wurde eine spezielle Type Polyamid 6 verwendet, welche keinerlei Gleitmittel enthält und somit ausschließlich die Wirkung der zu untersuchenden Additive ermittelt wurde. Diese Type ist aus diesem Grund ohne den nachträglichen Zusatz von Gleitmitteln nicht zu verarbeiten. Die Entformungskraft liegt > 10000 N.

Die Konzentrationen von Gleitmitteln mit dem besten Cost-Performance-Verhältnis liegen bei Polyamid üblicherweise bei ca. 0,3 phr (parts per hundred resin). Die verwendeten Gleitmittel und Additive wurden in das Polymergranulat eingemischt und mittels eines Doppelschneckenextruders eincompoundiert und anschließend nach Vortrocknung mittels Spritzguss verarbeitet.
Sämtliche Versuche wurden aufgrund der Vergleichbarkeit unter identischen Bedingungen (Temperaturprogramme, Schneckengeometrien, Spritzgießparameter etc.) durchgeführt.

### Beispiel 1

| Polyamid 6, unverstärkt | |
|---|---|
| 0,15 phr Nylostab S-EED* + 0,15 phr Calciummontanat | 400 N |

| Vergleich: | |
|---|---|
| Polyamid 6 ohne Gleitmittel | > 10 000 N |
| 0,3 phr Nylostab S-EED* | 1 500 N |
| 0,3 phr Calciummontanat | 550 N |
| 0,3 phr Montanwachsester | 550 N |

| | |
|---|---|
| (* Nylostab S-EED entspricht N,N'-bis(2,2,6,6-tetramethyl-4-piperidyl)-1,3-benzoldicarboxamid) | |

| Polyamid 6, glasfaserverstärkt | |
|---|---|
| 0,15 phr Nylostab S-EED + 0,15 phr Calciummontanat | 850 N |
| 0,15 phr Nylostab S-EED + 0,15 phr Montanwachsester | 800 N |

| Vergleich: | |
|---|---|
| Polyamid 6 (30 % Glasfaser) ohne Gleitmittel | 2500 N |
| 0,3 phr Calciummontanat | 1000 N |
| 0,3 phr Montanwachsester | 900 N |

Der alleinige Einsatz von jeweils Calciummontanat oder Montanwachsester entspricht dem Stand der Technik.

### Fließverbesserung von Polyamid (innere Gleitwirkung)

Gleitmittel werden in der Kunststoffindustrie als innere Gleitmittel eingesetzt um die Reibung der Polymerpartikel während des Verarbeitungsvorganges zu vermindern. Sie steigern somit die Fließfähigkeit des Polymers. Die Fließverbesserung (innere Gleitwirkung) von Gleitmitteln in technischen. Kunststoffen wie Polyamid wird üblicherweise durch die Bestimmung der Fließlänge mittels "Spiraltest" quantifiziert. Dazu wird eine skalierte Spirale im Spritzgießverfahren hergestellt und die Länge bestimmt. Ja länger der Fließweg (d.h. die Spirale), desto besser ist die innere Gleitwirkung, d.h. die Fließfähigkeit des Polymers.

Die verwendeten Gleitmittel und Additive wurden in das Polymergranulat eingemischt und mittels eines Doppelschneckenextruders eincompoundiert und anschließend nach Vortrocknung mittels Spritzguss verarbeitet. Sämtliche Versuche wurden aufgrund der Vergleichbarkeit unter konstanten Verarbeitungsbedingungen durchgeführt. Es wurde das spezielle Polyamid aus Beispiel 1 verwendet.

### Beispiel 2

| | |
|---|---|
| 0,15 phr Nylostab S-EED + 0,15 phr Calciummontanat | 42,0 cm |

| Vergleich: | |
|---|---|
| 0,3 phr Nylostab S-EED | 40,5 cm |
| 0,3 phr Calciummontanat | 41,0 cm |

Zusätzlich konnte diese Fließverbesserung von keinem der darüber hinaus getesteten Gleitmittel, welche nach dem Stand der Technik in Polyamiden eingesetzt werden, erreicht werden.

### Glanz von Polyamidcompounds

Gleitmittel beeinträchtigen sehr häufig den Glanz des Endartikels aus Polyamid. Angestrebt ist, mit den zugesetzten Gleitmitteln möglichst keinen negativen Einfluss auf den Glanz zu erhalten.

Die Glanzmessung erfolgt üblicherweise durch Bestimmung mit einem Reflektometer bei dem das reflektierte Licht unter einem bestimmten Einfallswinkel (Glanzwinkel) gemessen wird. Je höher die Glanzeinheiten desto höher ist der Glanz der Oberfläche. Gemäß DIN 67530 sollen Oberflächen, welche wie im vorliegenden Fall unter einem 60° Glanzwinkel Werte über 70 Einheiten zeigen unter einem Glanzwinkel von 20° gemessen werden. Die folgenden Werte ergaben sich bei Messung unter einem Glanzwinkel von 20°: Die Herstellung der Spritzplatten zur Glanzmessung erfolgte aufgrund der Vergleichbarkeit unter konstanten Bedingungen.

### Beispiel 3

| | |
|---|---|
| 0,15 phr Nylostab S-EED / 0,15 phr Calciummontanat | 89 Glanzeinheiten |

| Vergleich: | |
|---|---|
| Polyamid 6 ohne Gleitmittel | 87 Glanzeinheiten |
| 0,3 phr Nylostab S-EED | 65 Glanzeinheiten |
| 0,3 phr Calciummontanat | 70 Glanzeinheiten |

### Dispergierung von Pigmenten in Polyamiden

Stand der Technik ist es, zum Einfärben von Polyamid mit organischen Pigmenten sogenannte Masterbatche zu verwenden, welche Farbkonzentrate darstellen, die Polyamid als Trägermaterial besitzen. Für eine gute Dispergierung der Pigmente werden gemäß dem Stand der Technik Wachse als Dispergierhilfsmittel verwendet um eine gute Dispergierung und damit optimale Farbausbeute zu erreichen.

Speziell bei Materbatchen mit technischen Kunststoffen. wie Polyamid, werden Montanwachse als Dispergierhilfsmittel zur Benetzung der Pigmente eingesetzt, um die zur Agglomeration neigenden und schwer dispergierbaren Pigmente zu zerteilen. Quantifiziert wird dies durch den sogenannten Filterdrucktest, bei dem der Druckaufbau vor einem Filter bestimmter Maschenweite gemessen wird, welcher sich verstärkt aufbaut, je größer die Agglomerate sind, und somit den Filter zusetzten. Je besser dispergiert die Pigmente sind, desto besser können sie den Filter passieren und desto besser ist die Dispergierung. Somit ist ein kleiner Filterdruckwert gemessen in [bar/g Pigment] ein Maß für gute Dispergierwirkung der Gleitmittel.

Als Pigment wurde PV Echtrosa ausgewählt, da es in Polyamid schwer dispergierbar ist. Als Träger wurde ein additivfreies Polyamid 6 verwendet, um d alleinige Wirkung der zugesetzten Additive untersuchen zu können. Dabei wurde überraschend festgestellt, dass die erfindungsgemäße Kombinationen aus Montanwachsen wie Montanwachsester oder Calciummontanat mit Nylostab S-EED extrem gute Dispergierung, d.h. niedrige Filterdruckwerte liefert, welche durch keine andere Kombination erreicht werden konnte.

Die Pigmente und Additive wurden mittels Kaltmischung eingemischt und an einem Doppelschneckenextruder eincompoundiert. Anschließend erfolgte die Ermittlung des Druckfilterwertes über ein 14 µm Filter.

Folgende Rezepturen wurde verwendet und Filterdruckwerte bestimmt:

### Beispiel 4

| | | | | | | | | |
|---|---|---|---|---|---|---|---|---|
| Polyamid 6 unverstärkt | 60 % | 60 % | 60 % | 65 % | 65 % | 65 % | 65 % | 65 % |
| PV Echtrosa E | 30 % | 30 % | 30 % | 30 % | 30 % | 30 % | 30 % | 30% |
| Nylostab S-EED | 5 % | 5 % | | | | | | |
| Calciummontanat | 5 % | | 10 % | | | | | |
| Montanwachsester | | 5 % | | 5 % | | | | |
| Licowax OP | | | | | 5 % | | | |
| Licolub WE 40 | | | | | | 5 % | | |
| Licolub WE 4 | | | | | | | 5 % | |
| Ceridust® 5551 | | | | | | | | 5 % |
| Filterdruckwert [bar/g Pigment] | 4,0 | 3,6 | 18,0 | 6,0 | 11,4 | 13,4 | 12,0 | 16,6 |

Alle eingesetzten Wachse in vergleichbarer Konzentration lieferten im Vergleich zu der neu gefundenen Additivkombination schlechtere Filterwerte und damit schlechtere Dispergierwirkung.

## Patentansprüche

1. Mischung aus einem Wachs (Komponente A) und einem Polymeradditiv (Komponente B), **dadurch gekennzeichnet, dass** es sich bei der Komponente A um Umsetzungsprodukte von Montanwachssäuren mit Ethylenglykol und/oder um Umsetzungsprodukte von Montanwachssäuren mit einem Calciumsalz und es sich bei der Komponente B um N,N'-bis-pyperidyl-1,3-benzoldicarboxamid oder um N,N'-bis(2,2,6,6-tetramethyl-4-piperidyl)-1,3-benzoldicarboxyamid handelt und wobei die Mischung 10 bis 90 Gew.-% an Komponente A und 90 bis 10 Gew.-% an Komponente B enthält.

2. Mischung nach Anspruch 1, **dadurch gekennzeichnet, dass** es sich bei den Umsetzungsprodukten um eine Mischung aus Ethylenglykol-Mono-Montanwachssäureester, Ethylenglykol-dimontanwachs-säureester, Montanwachssäuren und Ethylenglykol handelt.

3. Mischung nach Anspruch 1, **dadurch gekennzeichnet, dass** es sich bei den Umsetzungsprodukten um eine Mischung aus Mischung aus 1,3-Budandiol-Mono-Montanwachssäureester, 1,3-Budandiol-Di-Montanwachssäureester, Montanwachssäuren, 1,3-Butandiol, Calciummontanat und dem Calciumsalz handelt.

4. Mischung nach einem oder mehreren der Ansprüche 1 bis 3, **dadurch gekennzeichnet, dass** sie 30 - 70 Gew.-% an Komponente A und 70 bis 30 Gew.-% an Komponente B enthält.

5. Mischung nach einem oder mehreren der Ansprüche 1 bis 4, **dadurch gekennzeichnet, dass** sie 45 bis 55 Gew.-% an Komponente A und 55 bis 45 Gew.-% an Komponente B enthält.

6. Mischung nach einem oder mehreren der Ansprüche 1 bis 5, **dadurch gekennzeichnet, dass** die Komponenten A und B als Granulat, Schuppen, Feinkorn, Pulver und/oder Micronisat vorliegen.

7. Mischung nach einem oder mehreren der Ansprüche 1 bis 6, **dadurch gekennzeichnet, dass** die Komponenten A und B, als physikalische Mischung der Feststoffe, als Schmelzmischung, als Kompaktat oder in Form eines Masterbatches vorliegen.

8. Verwendung einer Mischung nach einem oder mehreren der Ansprüche 1 bis 7 als Gleit-, Trenn- und/oder Dispergiermittel.

9. Verwendung einer Mischung nach Anspruch 8 in einer Formmasse eines Polykondensats.

10. Verwendung nach Anspruch 8 oder 9, **dadurch gekennzeichnet, dass** es sich bei dem Polykondensat um Polyamide handelt.

11. Verwendung nach einem oder mehreren der Ansprüche 8 bis 10, **dadurch gekennzeichnet, dass** es sich bei den Polyamiden um solche vom Aminosäure-Typ und/oder vom Diamin-Dicarbonsäure-Typ handelt.

12. Verwendung nach einem oder mehreren der Ansprüche 8 bis 11, **dadurch gekennzeichnet, dass** es sich bei den Polyamiden um Polyamid 6 und/oder Polyamid 66 handelt.

13. Verwendung nach einem oder mehreren der Ansprüche 8 bis 12, **dadurch gekennzeichnet, dass** die Polyamide unverändert, gefärbt, gefüllt, ungefüllt, verstärkt, unverstärkt oder auch anders modifiziert sind.

14. Verwendung nach einem oder mehreren der Ansprüche 8 bis 13, **dadurch gekennzeichnet, dass** die Komponenten A und B an den gleichen oder unterschiedlichen Prozessschritten bei der Herstellung/Verarbeitung von Polyamiden eingebracht werden.

15. Verwendung nach einem oder mehreren der Ansprüche 8 bis 14, **dadurch gekennzeichnet, dass** die Einarbeitung der Komponente A und B in der Polykondensation, in der Compoundierung oder direkt bei der formgebenden Verarbeitung erfolgt.

16. Verwendung nach einem oder mehreren der Ansprüche 8 bis 15, **dadurch gekennzeichnet, dass** die Gesamtmenge der Komponenten A und B 0,01 bis 10,00 Gew.-% im Polykondensat beträgt.

17. Verwendung nach einem oder mehreren der Ansprüche 8 bis 16, **dadurch gekennzeichnet, dass** die Gesamtmenge der Komponenten A und B 0,1 bis 2,00 Gew.-% im Polykondensat beträgt.

## Claims

1. A blend of a wax (component A) and a polymer additive (component B), wherein component A is products of the reaction of montan wax acids with ethylene glycol and/or is products of the reaction of montan wax acids with a calcium salt, and component B is N,N'-bis-piperidyl-1,3-benzenedicarboxamide or is N,N'-bis(2,2,6,6-tetramethyl-4-piperidyl)-1,3-benzenedicarboxyamide, and where the blend comprises from 10 to 90% by weight of component A and from 90 to 10% by weight of component B.

2. The blend as claimed in claim 1, wherein the products of the reaction are a mixture of the mono(montan wax acid) ester of ethylene glycol, the di(montan wax acid) ester of ethylene glycol, montan wax acids, and ethylene glycol.

3. The blend as claimed in claim 1, wherein the products of the reaction are a mixture of the mono(montan wax acid) ester of 1,3-butanediol, the di(montan wax acid) ester of 1,3-butanediol, montan wax acids, 1,3-butanediol, calcium montanate, and the calcium salt.

4. The blend as claimed in one or more of claims 1 to 3, which comprises from 30 to 70% by weight of component A and from 70 to 30% by weight of component B.

5. The blend as claimed in one or more of claims 1 to 4, which comprises from 45 to 55% by weight of component A and from 55 to 45% by weight of component B.

6. The blend as claimed in one or more of claims 1 to 5, wherein components A and B take the form of pellets, flakes, fine particles, powders, and/or micronizate.

7. The blend as claimed in one or more of claims 1 to 6, wherein components A and B take the form of a physical mixture of the solids, a melt mixture, a compactate, or a masterbatch.

8. The use of the blend as claimed in one or more of claims 1 to 7 as lubricant, release agent, and/or dispersing agent.

9. The use of the blend as claimed in claim 8 in a molding composition of a polycondensate.

10. The use as claimed in claim 8 or 9, wherein the polycondensate is polyamide.

11. The use as claimed in one or more of claims 8 to 10, wherein the polyamides are those of amino-acid type and/or those of diaminedicarboxylic-acid type.

12. The use as claimed in one or more of claims 8 to 11, wherein the polyamides are nylon-6 and/or nylon-6,6.

13. The use as claimed in one or more of claims 8 to 12, wherein the polyamides are unmodified, colored, filled, unfilled, reinforced, or unreinforced polyamides, or else polyamides which have been otherwise modified.

14. The use as claimed in one or more of claims 8 to 13, wherein components A and B are introduced in the same or different steps of the process during the preparation/processing of polyamides.

15. The use as claimed in one or more of claims 8 to 14, wherein components A and B are introduced during the polycondensation., during the compounding process, or directly during the shaping process.

16. The use as claimed in one or more of claims 8 to 15, wherein the total amount of components A and B is from 0.01 to 10.00% by weight in the polycondensate.

17. The use as claimed in one or more of claims 8 to 16, wherein the total amount of components A and B is from 0.1 to 2.00% by weight in the polycondensate.

## Revendications

1. Mélange d'une cire (Composant A) et d'un additif pour polymère (Composant B), **caractérisé en ce que**, pour ce qui concerne le Composant A, il s'agit de produits de la réaction d'acides montaniques avec de l'éthylèneglycol et/ou de produits de la réaction d'acides montaniques avec un sel de calcium et que, pour ce qui concerne le Composant B, il s'agit de N,N'-bis-pipéridyl-1,3-benzène-dicarboxamide ou de N,N'-bis(2,2,6,6-tétraméthyl-4-pipéridyl)-1,3-benzènedicarboxamide, le mélange contenant de 10 à 90 % en poids du Composant A et de 90 à 10 % en poids du Composant B.

2. Mélange selon la revendication 1, **caractérisé en ce que**, pour ce qui concerne les produits de réaction, il s'agit d'un mélange d'esters d'acide monomontanique de l'éthylèneglycol, d'esters d'acide dimontanique de l'éthylèneglycol, d'acides montaniques et d'éthylèneglycol.

3. Mélange selon la revendication 1, **caractérisé en ce que**, pour ce qui concerne les produits de réaction, il s'agit d'un mélange constitué d'un mélange de l'ester de l'acide monomontanique du 1,3-butanediol, de l'ester de l'acide dimontanique du 1,3-butanediol, d'acides montaniques, du 1,3-butanediol, du montanate de calcium et du sel de calcium.

4. Mélange selon l'une ou plusieurs des revendications 1 à 3, **caractérisé en ce qu'**il contient 30 à 70 % en poids du Composant A et 70 à 30 % en poids du Composant B.

5. Mélange selon l'une ou plusieurs des revendications 1 à 4, **caractérisé en ce qu'**il contient 45 à 55 % en poids du Composant A et 55 à 45 % en poids du Composant B.

6. Mélange selon l'une ou plusieurs des revendications 1 à 5, **caractérisé en ce que** les Composants A et B se présentent sous forme de granulés, d'écailles, de grains à fine granulométrie, d'une poudre et/ou d'un micronisat.

7. Mélange selon l'une ou plusieurs des revendications 1 à 6, **caractérisé en ce que** les Composants A et B se présentent sous forme d'un mélange physique des matières solides, d'un mélange à l'état fondu, d'un produit obtenu par compactage, ou sous forme d'un mélange maître.

8. Utilisation d'un mélange selon l'une ou plusieurs des revendications 1 à 7, en tant que lubrifiant, agent de démoulage et/ou dispersant.

9. Utilisation d'un mélange selon la revendication 8 sous forme d'un mélange à mouler d'un produit de polycondensation.

10. Utilisation selon la revendication 8 ou 9, **caractérisée en ce que**, pour ce qui concerne le produit de polycondensation, il s'agit de polyamides.

11. Utilisation selon l'une ou plusieurs des revendications 8 à 10, **caractérisée en ce que**, pour ce qui concerne les polyamides, il s'agit de ceux du type acide aminé et/ou du type acide diaminodicarboxylique.

12. Utilisation selon l'une ou plusieurs des revendications 8 à 11, **caractérisée en ce que**, pour ce qui concerne les polyamides, il s'agit du polyamide 6 et/ou du polyamide 66.

13. Utilisation selon l'une ou plusieurs des revendications 8 à 12, **caractérisée en ce que** les polyamides sont non modifiés, colorés, chargés, non chargés, renforcés, non renforcés ou aussi autrement modifiés.

14. Utilisation selon l'une ou plusieurs des revendications 8 à 13, **caractérisée en ce que** les Composants A et B sont introduits dans des étapes du procédé identiques ou différentes lors de la préparation/mise en oeuvre de polyamides.

15. Utilisation selon l'une ou plusieurs des revendications 8 à 14, **caractérisée en ce que** l'incorporation des Composants A et B est réalisée lors de la polycondensation, du mélangeage, ou directement lors de la mise en oeuvre formant façonnage.

16. Utilisation selon l'une ou plusieurs des revendications 8 à 15, **caractérisée en ce que** la quantité totale des Composants A et B est de 0,01 à 10, 00 % en poids dans le produit de polycondensation.

17. Utilisation selon l'une ou plusieurs des revendications 8 à 16, **caractérisée en ce que** la quantité totale des Composants A et B est de 0,1 à 2,00 % en poids dans le produit de polycondensation.
